# EUROPEAN PATENT SPECIFICATION

(11) **EP 1 575 663 B1**
(45) Date of publication and mention of the grant of the patent: **27.02.2008**
(21) Application number: 03753393.2
(22) Date of filing: 08.09.2003
(51) Int. Cl.: A61N 1/36, A61N 1/18

(54) **ELECTRICAL STIMULATOR**
ELEKTRISCHER STIMULATOR
STIMULATEUR ELECTRIQUE

(30) Priority: 19.12.2002 IT PD20020084 U
(43) Date of publication of application: 21.09.2005
(73) Proprietor: Struttura S.R.L., 36100 Vicenza (IT)
(72) Inventor: MORETTO, Maurizio, I-36100 Vicenza (IT)
(74) Representative: Montevecchi, Emma
(86) International application number: PCT/EP2003/009935
(87) International publication number: WO 2004/056419

(56) References cited:
- EP-A- 0 689 855
- EP-A- 0 706 806
- US-A- 5 620 483
- US-A1- 2002 165 590

## Description

### Technical field of the invention

The present invention relates to an electrical stimulator of the type including the characteristics mentioned in the preamble to the main claim.

### Background Art

Electrical stimulators are devices which can generate electrical pulses, typically square-wave pulses which, when appiied by means of electrodes to particular points of the human or animal body, induce a stress in selected muscle groups. Typically, pulsed contraction of one or more muscles is brought about with the purpose of massaging the body area concerned, toning, muscle strengthening, rehabilitation, training, etc.

The pulses and the parameters of the wave which produces them are controlled (directly or by means of pre-installed programs), according to the specific purposes of the desired treatment, by altering the frequency, the amplitude, the length and the shape of the wave and/or its intensity, alternation with rest periods, duration of application, etc.

In spite of all of these control capabilities, electrical stimulators that have been known up to now do not permit effective monitoring of the energy expenditure resulting from the muscle contractions which are stimulated. In other technical fields also relating to equipment for the physical exercise of muscles and for improving muscle tone, it is known to implement such functions by measuring the work performed by the muscle group concerned by measuring the energy produced. Typically, for example, in exercise cycles, dynamometric brakes or the like are used. A disadvantage of such measurements is that the measurement of the work performed is a total measurement and cannot be measured individually for each of the various muscle groups involved in the exercise (for example, the total work performed whilst pedalling for a predetermined period of time with predetermined braking adjustments is measured).

Electrical stimulators comprising the features of the preamble of claim 1 are disclosed in US patent application 2002/0165590.

### Disclosure of the Invention

The invention proposes as its main object that of making available an electrical stimulator which, in addition to the conventional adjustments and display of information, enables information on the work performed by muscle groups concerned to be displayed and/or processed, and/or permits adjustments functionally subject thereto.

A further object of the Invention is to provide these displays and/or adjustments in the form of calorie consumption.

These objects and yet others which will become clearer from the following description are achieved by an electrical stimulator formed In accordance with the appended claims.

### Brief description of the drawings

The characteristics and the advantages of the invention will become clearer from the description of a preferred embodiment thereof which is described with reference to the appended drawings, in which:

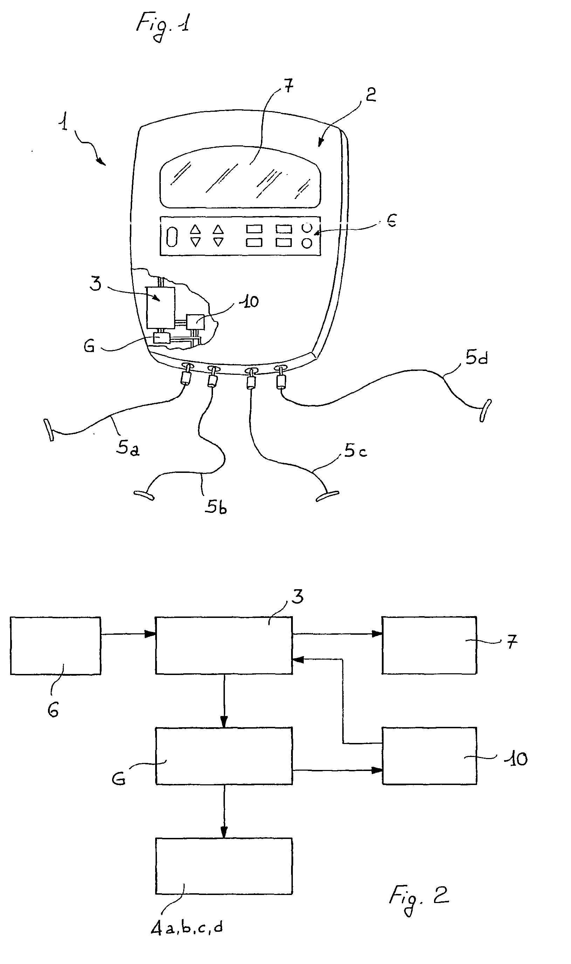

Figure 1 is a schematic view of an electrical stimulator formed in accordance with the present invention,
Figure 2 is a functional diagram indicating an operative characteristic of the electrical stimulator of Figure 1.

### Best mode for carrying out the invention

In the drawings, an electrical stimulator formed in accordance with the present invention is generally indicated 1. The electrical stimulator 1 comprises, in conventional manner, a housing 2 containing a circuit board 3. The board 3 comprises, In known manner, a multi-channel pulse-generator G in which each channel Is associated with a respective plug connector 4a, 4b, 4c, 4d for connecting the electrical stimulator 1 to the user's body by means of electrodes and respective connecting wires, generally indicated 5a, 5b, 5c, 5d. Each pulse channel is adjustable in intensity by means of an adjuster represented in the drawing by a "+" button and a "-" button forming part of a keypad 6 for the programming and control of the electrical stimulator. A display 7 is provided for displaying the programming and operational parameters of the device. The keypad 6 and the display 7 are connected to the board 3.

According to the invention, counting and processing means 10 are also provided on the board 3 or by means of a remote connection and are associated with the board and with the pulse generator G in a manner such as to detect the number and type of pulses generated over time and to calculate the resulting muscle work and the consequent calorie consumption. In particular, the counting and processing means 10 are provided, for example, by means of the keypad 6, with the following parameters: the muscles concerned, parameters relating to the individual pulses for each muscle and/or muscle group, and the duration and cyclical characteristics of the train of pulses.

Data relating to the instrument such as, for example, the wave frequency, the amplitude, the duration, the intensity and the sequence of the trains of pulses and of the pause times, etc. can be derived at least partially from the predefined parameters relating to the electrical stimulation program in progress. Other parameters, on the other hand, such as personal body data (e.g. age, sex, weight, height, body mass index, ...) or the muscle area concerned (e.g. biceps, abdominals, quadriceps, trapezius, ...), or the period of use (e.g. 30 minutes twice a day, or one hour continuously,...) can be defined by the user. Some parameters can be added or removed so as to determine and optimise the calculation of the calories burned and/or relative counts functionally associated with this determination (calories burned per unit of time, total calories expended, or target for calories to be burned, in order to define the period of use, ...) according to the desired precision.

By virtue of the counting and processing means 10, it is possible to display on the display 7 the sum of the calories used during the period of application, that is, the sum extended to several time stages (daily consumption, consumption over several days, weekly consumption, monthly consumption, etc.) that is, the quantity of calories to be burned by the various applications, or even the countdown of calories remaining to be used for each individual application or, finally, the daily calorie consumption for a "weight/fitness" programme by which it is desired to conform to a training and/or slimming programme.

For this purpose, the processing means are arranged to sum the value indicative of the calorie expenditure associated with the pulses, integrated over the period of application of the pulses.

As stated, the counting and processing means may be incorporated structurally in the same housing with the electrical stimulator or may be associated therewith externally.

The following are the main advantages of the invention:
- the ability to check the calorie expenditure correlated with a specific exercise in real time,
- the ability to create exercise tables aimed at each part of the body for specific localized slimming or for generalized slimming,
- the ability to create a work table in order to establish a series of exercises beforehand by checking their calorie expenditure and then confirming their efficacy in the patient,
- the ability to select the parts of the body to be subjected to electrical stimulation and the period of action in dependence on the preselected calorie-consumption target,
- an improved capability to correlate the electrical stimulation activity with dietary programmes.

## Claims

1. An electrical stimulator including an electrical-pulse generator (G) which can be connected to a user by means of one or more electrodes (5a, 5b, 5c, 5d), **characterized in that** it comprises means (10) for counting the pulses and means (10) for processing the count in order to convert a value indicative of the count into a value indicative of the calorie expenditure associated with the pulses.

2. An electrical stimulator according to Claim 1 in which the processing means (10) are arranged to sum the said value indicative of the calorie expenditure, integrated over the period of application of the pulses.

3. An electrical stimulator according to Claim 1 or Claim 2 in which the counting and processing means (10) are structurally incorporated in the same housing (2) with the electrical stimulator.

## Patentansprüche

1. Elektrische Anregungseinrichtung, die einen elektrischen Impulsgenerator (G) umfasst, der mittels einer oder mehrerer Elektroden (5a, 5b, 5c, 5d) mit einem Nutzer verbunden werden kann,
**dadurch gekennzeichnet, dass** sie Mittel (10) zum Zählen der Impulse und Mittel (10) zum Verarbeiten der Zählung umfasst, um einen für die Zählung indikativen Wert in einen Wert umzuwandeln, der für den Kalorienverbrauch indikativ ist, der mit den Impulsen assoziiert ist.

2. Elektrische Anregungseinrichtung gemäß Anspruch 1, wobei die Verarbeitungsmittel (10) ausgelegt sind, den für den Kalorienverbrauch indikativen Wert integriert über die Aufbringungszeitdauer der Impulse aufzusummieren.

3. Elektrische Anregungseinrichtung gemäß Anspruch 1 oder Anspruch 2, wobei die Zählungs- und Verarbeitungsmittel (10) strukturell in demselben Gehäuse (2) mit der elektrischen Anregungseinrichtung untergebracht sind.

## Revendications

1. Stimulateur électrique comprenant un générateur d'impulsions électriques (G) qui peut être relié à un utilisateur au moyen d'une ou de plusieurs électrodes (5a, 5b, 5c, 5d), **caractérisé en ce qu'**il comprend des moyens (10) pour compter les impulsions, et des moyens (10) pour traiter le comptage afin de convertir une valeur indicative du comptage en une valeur indicative de la dépense calorique associée aux impulsions.

2. Stimulateur électrique selon la revendication 1, dans lequel les moyens de traitement (10) sont configurés pour additionner ladite valeur indicative de la dépense calorique intégrée sur la période d'application des impulsions.

3. Stimulateur électrique selon la revendication 1 ou 2, dans lequel les moyens de comptage et de traitement (10) sont incorporés structurellement dans le même boîtier (2) que le stimulateur électrique.
